# EUROPÄISCHE PATENTSCHRIFT

(11) **EP 1 755 569 B1**
(45) Veröffentlichungstag und Bekanntmachung des Hinweises auf die Patenterteilung: **03.08.2011**
(21) Anmeldenummer: 05748424.8
(22) Anmeldetag: 10.06.2005
(51) Int. Cl.: A61K 9/70, A61L 15/18, A61L 15/34

(54) **WUNDAUFLAGE**
WOUND DRESSING
PANSEMENT

(30) Priorität: 24.06.2004 DE 102004031955
(43) Veröffentlichungstag der Anmeldung: 28.02.2007
(73) Patentinhaber: Paul Hartmann AG, 89522 Heidenheim (DE)
(72) Erfinder: GOERL, Rudolf, 63785 Obernburg (DE); EFFING, Jochem, 65779 Kelkheim-Fischbach (DE)
(74) Vertreter: Dreiss
(86) Internationale Anmeldenummer: PCT/EP2005/006236
(87) Internationale Veröffentlichungsnummer: WO 2006/000304

(56) Entgegenhaltungen:
- EP-A- 1 120 112
- EP-A- 1 159 972
- WO-A-2004/112852
- US-A- 6 087 549

## Beschreibung

Die Erfindung betrifft eine Wundauflage umfassend einen Träger mit einer ersten einer zu behandelnden Oberfläche zugewandten Seite und einer zweiten gegenüberliegenden Seite, der mit einem antimikrobiell wirkenden Metall ausgerüstet ist.

Derartige Wundauflagen werden beispielsweise zur Behandlung infizierter Wunden oder zum vorbeugenden Schutz vor Wundinfektionen eingesetzt. Die Behandlung von infizierten bzw. kontaminierten Wunden stellt die Medizin häufig vor Probleme. Dies gilt insbesondere für schwer heilende und chronische Wunden, wobei der Verlauf der Heilung durch die Besiedlung mit Keimen stark verzögert wird. Aber auch bei akuten Verletzungen stellt eine Keimbesiedlung ein großes Risiko dar. Es gibt daher im Stand der Technik eine Vielzahl von Ansätzen, Mikroorganismen aus besiedelten und infizierten Wunden zu entfernen bzw. darin abzutöten und/oder eine Besiedlung zu vermeiden. Außer der oralen Gabe von Antibiotika kann das Entfernen pathogener Mikroorganismen durch die topische Anwendung von Desinfektionsmitteln oder Antibiotika erreicht werden. Allerdings sind viele Antiseptika zytotoxisch. Darüber hinaus bestehen häufig Resistenzen pathogener Keime gegen Antibiotika.

Es ist daher seit langem im Stand der Technik bekannt, zur Verwendung bei infizierten Wunden antimikrobiell wirkende Metalle und Oxidanzien z.B. Jodtinktur einzusetzen.

So ist es beispielsweise aus der DE 199 58 458 A1 bekannt, eine Wundauflage mit antimikrobiellen Eigenschaften vorzusehen, bei der selbstklebende oder nicht selbstklebende Polymer-Materialien, die in der Wundheilung Verwendung finden, kombiniert werden mit silberhaltigen Zeolithen, die in die Polymermaterialien eingearbeitet werden können. Dabei wirken die Zeolithe als Ionentauscher, wobei geringe, definierte Mengen als Silberionen im feuchten Milieu freigesetzt werden und hierdurch eine langanhaltende antibakterielle Wirkung garantiert werden soll.

Des Weiteren ist es aus der DE 30 33 606 bekannt, einen antiadhäsiven, antimikrobiellen Wundverband auf der Basis von Verbandstoffen und pharmazeutischen Zubereitungen einzusetzen, bei dem ein antiadhäsives oder antiadhäsiv ausgerüstetes Gewebe oder Vlies aus natürlichen oder synthetischen Fasern mit einer antimikrobiellen Zubereitung, beispielsweise Silbersulfadiazin-Salbe beschichtet ist und zur lokalen Therapie verwandt wird.

Darüber hinaus ist es aus der WO 01/60599 A1 bekannt, ein mehrschichtiges Laminat bereitzustellen, mit einer ersten Schicht aus einem Polymermaterial und einer zweiten Schicht, die mit der ersten Schicht verbunden ist, wobei die zweite Schicht ein bakteriozides Metall enthält, wobei das Metall vorzugsweise Silber sein kann. Nachteilig bei dieser Gestaltung ist, dass die Freisetzung des Metalls beim Kontakt mit dem feuchten Milieu der Wundoberfläche nicht gesteuert werden kann und ggf. eine zu große frühzeitige Abgabe einer Metallmenge erfolgt, durch die gesundheitliche Beeinträchtigungen gegeben sein können und wodurch ein frühzeitiges Ersetzen des Verbandes notwendig wird.

Des Weiteren offenbart die WO 2004/007595 A1 ein Schaummaterial, bei dem das Schaummaterial eine antimikrobielle Komponente umfasst, die in kontrollierter Menge abgegeben werden soll.

Des Weiteren zeigt die WO 2004/002384 A1 ein Verbandmaterial mit antibakterieller, antiviraler und/oder antifungizider Aktivität. Wobei auch hier eine kontrollierte Abgabe von ionischem Silber in die Wundflüssigkeit erfolgen soll, wobei das Verbandmaterial aus einem hydrophilen, anionischen Polymer besteht, an das vorzugsweise Silber über eine polare oder ionische Bindung gebunden ist.

Die WO 02/085387 A2 zeigt die Verwendung eines antimikrobiell wirkenden Metalls in Form von Nanokristallen.

Des Weiteren zeigt die US 6,087,548 einen mehrlagigen Wundverband, wobei hier sich Silberlagen oder silberbeschichtete Faserlagen in einem gewebten Textilmaterial mit solchen Lagen abwechseln, die vorzugsweise aus einem nicht metallischen, nicht leitenden Material bestehen. Dabei enthalten die einzelnen Schichten vorzugsweise verschiedene Anteile an Silber.

Schließlich beschreibt die WO 2004/112852 A1 einen Wundverband umfassend eine erste flüssigkeitsdurchlässige Schicht und eine zweite hierauf angeordnete Schicht, wobei ein antimikrobielles Metall in elementarer Form zwischen der ersten und der zweiten Schicht vorliegt und im Wesentlichen kein antimikrobielles Metall in elementarer Form auf der Außenseite der Oberflächen der zwei Lagen vorgesehen ist.

Ausgehend vom Stand der Technik ist es nun Aufgabe der Erfindung, eine Wundauflage in alternativer Form bereit zu stellen, bei der ein antimikrobiell wirkendes Metall kontrolliert abgegeben werden kann. Eine weitere Aufgabe der Erfindung ist es, ein Verfahren zur Herstellung einer antimikrobiell wirkenden Wundauflage bereit zu stellen, insbesondere zur Behandlung von mit pathologischen Keimen befallenen Wunden.

Die Erfindung löst diese Aufgabe durch eine Wundauflage umfassend einen Träger mit einer ersten einer zu behandelnden Oberfläche zugewandten Seite und eine zweiten gegenüberliegenden Seite, der mit einem antimikrobiell wirkenden Metall ausgerüstet ist, wobei der Träger mindestens auf seiner ersten Seite eine Zubereitung zur lokalen Therapie umfasst und wobei die Zubereitung eine Salbe oder Creme ist. Dass der Träger mindestens auf seiner ersten Seite eine Zubereitung umfasst ist dabei erfindungsgemäß so zu verstehen, dass dabei die Zubereitung entweder unmittelbar auf dem mit Metall versehenen Träger angeordnet ist oder zunächst auf der ersten Seite eine kontinuierliche oder diskontinuierliche Metallschicht angebracht ist, auf die dann wiederum die Zubereitung aufgebracht ist. Es kann auch ein beidseitiges Aufbringen der Zubereitung erwünscht sein, insbesondere wenn ein Eintamponieren der Wundauflage vorgenommen werden soll. In diesem Fall ist es vorteilhaft, wenn auch der Metallauftrag beidseitig oder das Gewirk umschließend aufgebracht ist.

Dabei wirkt die Zubereitung, die eine Salbe oder Creme ist, als Vermittler zwischen der mit dem Metall ausgerüsteten Wundauflage und der Wunde des Patienten. Auf diese Weise kann ein unmittelbarer Kontakt mit der Wundauflage und insbesondere auch ein Verkleben derselben mit der Wunde sicher vermieden werden. Darüber hinaus kann diese Salbe oder Creme im Bereich der Peri-Wundhaut eine pflegende Wirkung erzeugen. Tritt die Wundauflage dann über die Zubereitung mit der Wunde in Kontakt, wird insbesondere über die Wundflüssigkeit durch Vermittlung der Zubereitung das Metall, z. B. Silber aus der Wundauflage freigesetzt und gelangt über die Zubereitung in die Wunde. Hierbei kann über die Auswahl der Zubereitung die Dosis an freigesetztem Metall reguliert werden. Dabei kann insbesondere vorgesehen sein, dass als Metall elementares Silber eingesetzt wird. Das Metall kann als Beschichtung auf der Wundauflage bzw. auf dem Träger angeordnet sein oder in den Träger einimprägniert werden. Dabei kann es nach einer ersten Ausgestaltung auf der der Wunde zugewandten ersten Seite des Trägers angeordnet sein.

Als Träger kommen insbesondere Polyamidträgergewirke, insbesondere bestehend aus einem Nylon-Faden in Frage.

Um eine besonders gute Vermittlung über die Zubereitung zu erreichen, kann vorgesehen sein, dass die Zubereitung selbst eine hohe Wasseraufnahmefähigkeit besitzt. Auch ist von Vorteil, wenn die Zubereitung wasserfrei ist. Auf diese Weise kann eine Voroxidation des Metalls vor Verwendung der Wundauflage verhindert oder zumindest vermindert werden. Erst wenn die Wundauflage in Kontakt mit der Wunde selbst tritt und somit Flüssigkeit hinzugelangt, erfolgt eine Freisetzung der Metallionen. Auf diese Weise kann sichergestellt werden, dass die Wirksamkeit der Wundauflage während z. B. einer Lagerung derselben in einem definierten Bereich bleibt. Darüber hinaus sorgt eine erhöht Sekretabsonderung einer Wunde für eine erhöhte Freigabe des antimikrobiell wirkenden Metalls, was insoweit von Vorteil ist, als Wundflüssigkeit einen Nährboden für alle Art von Keimen bietet und in diesen Fällen eine erhöhte Abgabe des Wirkstoffs erfolgt.

Insbesondere kann vorgesehen sein, dass die Wundauflage weniger als 500 µg/100cm² in 24h bei 37°C in 100 ml Wasser an antimikrobiellem Material freisetzt, insbesondere weniger als 400 µg/100cm² in 24h bei 37°C in 100 ml Wasser und insbesondere weniger als 300 µg/100cm² in 24h bei 37°C in 100 ml Wasser freisetzt. Jedoch kann insbesondere vorgesehen sein, das die Wundauflage mehr als 2 µg/100cm² in 24h bei 37°C in 100 ml Wasser freisetzt.

Schließlich kann vorgesehen sein, dass die Zubereitung mindestens ein Mittel ausgewählt aus der Gruppe der Mono-, Di- oder Triglyceride, Fettsäureester Oligomere des Glycerin, Fettalkohole, Fettsäureester, ethoxylierten Fettalkohole, ethoxylierten Fettsäuren, Polyethoxylen-Derivate oder dimerisierten Fettsäureester oder Derivate hiervon umfasst. Insbesondere kann die Zubereitung ein Gemisch umfassend ein Gemisch aus verschiedenen Mono-, Di- und Triglyceriden und/oder Fettsäureester Oligomerer des Glycerins und/oder Polyethylenglycolen sein. Ganz besonders vorteilhaft ist eine Zubereitung, die 1 - 80 Gew.-% an Mono-, Di- und Triglyceriden, 1 - 80 Gew.-% Fettsäureester Oligomerer des Glycerins und 1 - 30 Gew.-% Polyethylenglycol umfasst.

In einer weiteren vorteilhaften Ausführung kann der Zubereitung ein wasserbindendes Mittel aus der Gruppe der Biopolymere ausgewählt aus der Gruppe der Alginate, Carboxymethylcellulose oder Chitosan zugesetzt sein. Dabei ist es besonders vorteilhaft 1 - 30 Gew.-% an wasserbindendem Mittel der Zubereitung zuzusetzen.

In einer weiteren Ausführung kann vorgesehen sein, dass die Zubereitung dimerisierte Fettsäureester oder Derivate hiervon und ein wasserbindendes Mittel umfasst. Hierbei kann insbesondere vorgesehen sein, dass die Zubereitung 1 - 99 Gew.-% dimerisierte Fettsäureester oder Derivate hiervon und 1 - 30 Gew.-% wasserbindende Mittel umfasst.

Besonders vorteilhaft ist ein Auftrag einer Zusammensetzung in Form einer Salbe oder Creme in einer Menge von mindestens 50 g/ m² Trägermaterial, insbesondere von mindestens 100 g/ m² Trägermaterial und ganz besonders bevorzugt von 100 bis 250 g/ m² Trägermaterial vorzusehen.

Nach einer besonders bevorzugten Ausführungsform, kann vorgesehen sein, dass der Träger mehrschichtig ist. In diesem Fall kann vorgesehen sein, dass lediglich eine der Schichten des Trägermaterials ein antimikrobielles Metall aufweist, das durch die Wundauflage freisetzbar ist. Alternativ können antimikrobielle Metalle gleicher oder verschiedener Art in mehreren Schichten untergebracht sein.

Schließlich kann auch vorgesehen sein, dass die Zubereitung nicht vollflächig aufgetragen ist, sondern einzelne Bereiche der Wundauflage ausgespart sind, beispielsweise um hier einen Klebeauftrag zur Befestigung der Wundauflage auf der Haut eines Patienten anzubringen. Alternativ kann jedoch die Wundauflage auch vollflächig bzw. beidseits mit der Zubereitung versehen sein, insbesondere, wenn die Wundauflage für größere zu behandelnde Wunden und Oberflächen eingesetzt werden soll bzw. ein Eintamponieren in eine Wunde erwünscht ist.

In einer weiteren Ausführung kann die erfindungsgemäße Wundauflage als primäre Wundauflage mit herkömmlichen Wundauflagen als sekundäre Wundauflage kombiniert werden. Hierbei können als sekundären Wundauflagen z.B. Wundauflagen aus hydrophilen Schäumen z.B. hydrophilen Polyurethanschäumen, Wundauflagen auf Alginatbasis z.B. faserförmige Alginate, Wundauflagen aus Hydrogelen z.B. Polyurethanharnstoffe, hydrocolloidale Wundauflagen oder Mull zum Einsatz gebracht werden. Bei dieser Kombination ist die sekundäre Wundauflage auf der zweiten Seite der erfindungsgemäßen Wundauflage aufgebracht. Als besonders günstig hat sich erwiesen, die erfindungsgemäße Wundauflage auf der zweiten Seite mit einem Folienverband, insbesondere ein atmungsaktiven Folienverband zu versehen. In einer ganz besonders bevorzugten Ausgestaltung ist die erfindungsgemäße Wundauflage auf der zweiten Seite mit einem selbstklebenden und atmungsaktiven Polyurethan Folienverband versehen.

Weiterhin betrifft die Erfindung ein Verfahren zur Herstellung einer antimikrobiellen Wundauflage bestehend aus den Schritten, Bereitstellen eines mit einem antimikrobiell wirkenden Metall ausgerüsteten Trägers und Aufbringen einer Zubereitung, nämlich einer Salbe oder Creme, auf mindestens eine Seite des Trägers. Dabei kann das Aufbringen vorzugsweise in Form einer Beschichtung oder Beaufschlagung erfolgen. Die Zubereitung kann dabei der vorstehend beschriebenen Zubereitung entsprechen.

Schließlich umfasst die Erfindung auch eine Verwendung einer Wundauflage umfassend einen mit einem antimikrobiell wirkenden Metall ausgerüsteten Träger und eine darauf aufgebrachte Zubereitung, insbesondere der vorstehend beschriebenen Art zur Herstellung eines Mittels zur Behandlung von Wunden.

Die Erfindung soll im Folgenden anhand von Ausführungsbeispielen näher erläutert werden.

Dabei wurde folgende Prüfmethode zur Bestimmung der Silberionenkonzentration herangezogen:
Die Probenvorbereitung zur Bestimmung der
Ionenfreisetzungsrate von metallisierten Trägermaterialien in Wasser erfolgte dabei wie folgt. Als Reagenzien werden bidestilliertes Wasser, 65% Salpetersäure (zur Analyse, Firma Merk) und aus der 65% Salpetersäure hergestellte, verdünnte Salpetersäure (2 mol/l) verwendet.

Die verwendeten Glasgeräte werden vor Gebrauch mit warmer, verdünnter Salpetersäure gereinigt, mit bidestilliertem Wasser gespült und getrocknet. Als Probenstück wird eine runde Scheibe mit 50 mm Durchmesser des zu untersuchenden Materials mittels eines Stanzmessers ausgestanzt und gewogen (Analysenwaage, Wägegenauigkeit d_{d}=0,1 mg) Dieses Probenstück wird flach auf den Boden eines Erlenmeyerkolbens (Schliffstopfen, NS 29) ausgelegt und mit 10 ml bidestilliertem Wasser (Vollpipette 10 ml) bedeckt.

Der Kolben wird verschlossen und für die Messzeit (24 Stunden) bei 37°C stehen gelassen. Nach dieser Zeit wird der Extrakt für die Analyse in ein Behältnis aus dunklem Glas oder dunklem Kunststoff dekantiert. Bei Bedarf wird der Extrakt zur Abtrennung von festen Bestandteilen zentrifugiert. Zur Stabilisierung werden nach dem Dekantieren und/oder Zentrifugieren 10 ml Extrakt 10 µl 65%-ige Salpetersäure zugesetzt. Unter den gleichen Bedingungen wird eine Blindwertlösung (ohne Probe) hergestellt. Die letztendliche Bestimmung des Silbergehalts erfolgt durch Atomabsorptionsspektroskopie gemäß DIN 38406-E18 (E18).

Zur Bestimmung der antimikrobiellen Aktivität wurde folgende Prüfmethode herangezogen: Die Bestimmung erfolgte gemäß der Standartmethode ASTM E 2180-01. Die Messungen wurden unter Verwendung von Ringer-Lösung an gram-positiven Keimen (Staphylococcus aureus) und gram-negativen Keimen (Staphylococcus aureus) durchgeführt. Je höher der bestimmte absolute Wert, desto aktiver ist die zur Untersuchung stehende Probe.

Bei der verwendeten Wundauflage handelt es sich um ein mit elementarem Silber beschichtetes Polyamidträgergewirk, das aus Nylon 6 Trilobalfäden mit 50 µm Kantenlänge hergestellt ist. Das Rohmaterial (ohne Versilberung) hat ein Flächengewicht von 25 bis 30 g/m² und eine Fadenfeinheit von dₜₑₓ= 22/1. In dem Material sind auf einem Quadratmeter zwischen 11.360 und 13.640 m Faden verwirkt. Dies bedeutet, dass die Oberfläche zwischen 1,7m² und 2,04m² liegt. Die Schichtdicke des Silbers auf dem Trägermaterial beträgt ca. 700 nm. Zwischen versilbertem und unversilbertem Produkt ist mit den herkömmlichen Mitteln (Lichtmikroskop) kein Unterschied in der Dreiecksseitenlänge feststellbar. Das Flächengewicht des versilberten Gewirks beläuft sich auf 35 +/- 3 g/m². Dabei ist Beträgt der Gehalt an elementarem Silber ca. 130 mg/ g Polyamidträgergewirk. Der Flächenwiderstand beträgt < 2/cm² (typischerweise zwischen 0,55 und 0,95 /cm²).

Derartige silberbeschichtete Gewirke werden von der Firma Statex, Bremen unter dem Namen Shieldex® Tüll Typ Charmeuse vertrieben und wurden hier als Trägermaterial eingesetzt.

Als Zubereitung wurden verschiedene Salbenmassen eingesetzt; die im Folgenden beschrieben sind:
Softisan 378 (Firma Sasol, Witten) : Hierbei handelt es sich um eine Handelsbezeichnung für ein Triglyceridgemisch einer Mischung von gesättigten geradzahligen Pflanzenfettsäuren (C8 bis C18) pflanzlichen Ursprungs (Caprylic/Capric/Staearic Triglyceride), das ein physiologisch unbedenkliches Produkt mit guter Haut und Schleimhautverträglichkeit darstellt und in wasserfreien Zubereitungen eingesetzt werden kann.

Softisan 649 (Firma Sasol, Witten) : Wollwachs-Austauschprodukt, Fettsäurester des oligomeren Glycerins (vorwiegend Diglycerin) CTFA-Bezeichnung: Caprylic/Capric/Isostearic/Adipic Triglyceride).

PEG xxx (Firma Clariant, Offenbach) : Polyethylenglycole verschiedener Kettenlängen, nicht ionogene Verbindungen aufgebaut aus Ethylenoxid-Einheiten MG = 44 (durchschnittliches Molekulargewicht); allgemeine Formel HOCH₂-(CH₂-O-CH₂)ₙ-CH₂-OH mit n = 4 - 70.

Lusplan (Firma Nippon Fine Chemicals, Osaka) : Dimerisierter Fettsäureester gemäß folgender Darstellung:

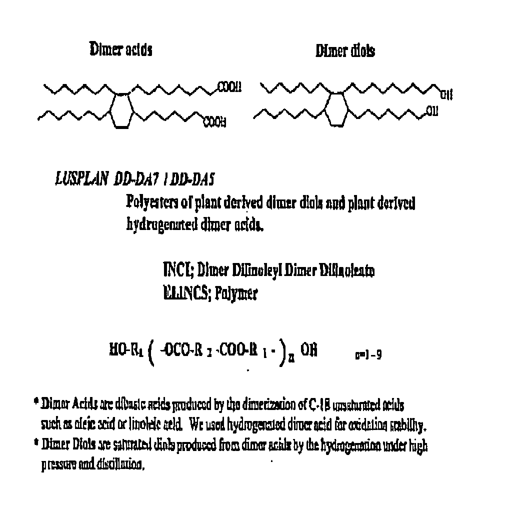

Es wurden Wundauflagen mit den verschiedenen Zubereitungen hergestellt, wobei die Ergebnisse der Tests in Bezug auf die Silberionenabgabe sowie auf die Keimreduktion in nachfolgender Tabelle dargestellt sind. Hierbei ist ein Auftrag der Salbenmasse in einer Menge von 185 g/ m² ± 20 % (bezogen auf das mit Silber beschichtete Trägermaterial erfolgt

Bei Beispiel 1 handelt es sich um ein Trägermaterial mit einer Versilberung, jedoch ohne aufgebrachte Salbenmasse. Für Beispiel 2 wurde das vorher bezeichnete Trägermaterial verwandt und hierauf eine Zubereitung bestehend aus 25 % Softisan 649 und 75% Softisan 378 aufgebracht. Die Muster 3 bis 6 betreffen Beschichtungen mit PEG verschiedener Kettenlänge, für Muster Nr. 7 wurde eine Beschichtung mit Lusplan 5 vorgenommen. Die Muster 8 bis 12 betreffen bezüglich der Salbenmasse Gemische aus Softisan sowie PEG in verschiedenen Varianten. Als Muster 13 wurde zu Softisan 649, Vaseline und Tecerowachs in den in der Tabelle genannten Anteilen hinzugegeben. Die Beispiele 14 bis 17 betreffen bereits auf dem Markt befindliche Produkte, wobei es sich bei dem Produkt Antisorbe® um ein Produkt der Firma Johnson & Johnson sowie bei Acticoat® um ein Produkt der Firma Smith & Nephew handelt.

Bezüglich aller genannten Proben 1 bis 17 wurde die Silberionenabgabe bezogen auf 24h und 37°C bezüglich 100 cm² Probenfläche nach dem beschriebenen Verfahren ermittelt. Die Darstellung in der Tabelle erfolgte einmal bezogen auf µg/100 ml Wasser bzw. µg/ml-Wasser.

Bei einer Wundauflage ohne Beschichtung mit einer Zubereitung, wie erfindungsgemäß vorgesehen, erfolgt eine Abgabe von Ionen im Bereich von 426,7 µg/100 ml Wasser.

Die Abgabe von Silberionen beim Produkt der Firma Smith & Nephew Acticoat® lag mit 9530,0 µg/100 ml Wasser am höchsten. Bei diesem Produkt handelt es sich um einen antimikrobiellen Wundverband, insbesondere gegen resistente Mikroorganismen wie Methicillin-resistente Staphylococcus aureus (MRSA) oder den Vancomycin-resistenten Enterococcus (VRE). Dabei wird beim Produkt Acticoat® eine Beschichtung eines Trägermaterials mit Nanokristallen vorgenommen.

Das Produkt Contreet H der Firma Coloplast weist eine Silberionenabgabe von 1330 µg/100 ml auf. Bei dem Produkt handelt es sich um einen Hydrocolloidverband, bei dem ionisches Silber eingesetzt wird. Auch hier erfolgt eine als nachteilig anzusehende sehr hohe Abgabe von Silberionen. Ebenfalls das Produkt der Firma Convatec Aquasell AG, bei dem es sich um eine absorbierende Wundauflage handelt, bei der ionisches Silber freigesetzt wird, weist eine hohe Silberabgabe auf, die deutlich über der Silberabgabe des Trägermaterials ohne Beschichtung, wie es erfindungsgemäß mit Beschichtung eingesetzt werden soll, liegt. Erfindungsgemäß ist jedoch erwünscht, dass die Abgabe an Silberionen unter 500 µg/100 cm² in 24h bei 37°C in 100 ml Wasser liegen soll.

Das Produkt Actisorb® der Firma Johnson & Johnson weist eine Silberionenabgabe von 40,6 µg/100 ml auf. Bei diesem Produkt handelt es sich um eine vliesstoffumhüllte Aktivkohle-Kompresse, die mit Silber bedampft ist. Diese Wundauflage ist nicht mit einer Zubereitung beaufschlagt, die eine gezielte Abgabe an Silberionen gewährleistet.

Der Tabelle kann dabei entnommen werden, dass sämtliche erfindungsgemäßen Gestaltungen dieses Kriterium erfüllen und eine Ionenabgabe insbesondere unter 350 mg/100 cm² aufweisen.

Des Weiteren wurde für eine Auswahl die Keimreduktion gemäß der beschriebenen Prüfmethode festgestellt. Die Keimreduktion wurde dabei einmal nach 4 und einmal nach 24 Stunden bestimmt, wobei hier die Darstellung in der üblichen logarithmischen Form erfolgt. Die ermittelten Werte für die Keimreduktion von z.B. gram-positiven Keimen von log 1,9 nach 4 Stunden bzw. von log 4,2 nach 24 Stunden für Beispiel 2 sowie log 1,6 nach 4 Stunden bzw. log 4,7 nach 24 Stunden für Beispiel 12 zeigen, das diese Beispiele eine gute antimikrobielle Wirkung besitzen. Auch nach 24 Stunden liegt die Keimreduktion im Bereich von log 4,6 für ein Trägermaterial ohne Beschichtung mit einer Salbenmasse ähnlich hoch. Damit kann gezeigt werden, dass mit Hilfe von vergleichsweise geringeren Silberabgaben, die geringere toxische Wirkungen auf den Patienten besitzen, ebenfalls gute Keimreduktionen erzielt werden. Darüber hinaus kann durch Hinzufügung der Salbemasse eine Abgabe von Silberionen über einen längeren Zeitraum erfolgen. Die Abgabe von Silberionen erfolgt hierbei in der gewünschten kontrollierten Form, nach Zutritt von Flüssigkeit durch Freisetzung von Ionen.

| Nr. | Trägermaterial beschichtet mit Salbenmasse bestebend aus : | Silberionenabgabe bezogen auf 24h, 37° C, 100 cm² Probenfläche | | Keimreduktion ASTH 2180 gram-positiv | | Keimreduktion ASTM 2180 Gram-negativ | |
|---|---|---|---|---|---|---|---|
| | | µg/ 100 ml Wasser | µg/ ml Wasser | log10 in 4 h | log10 in 24 h | log10 in 4 h | log10 in 24 h |
| | 1 ohne Beschichtung | 426,7 | 4,2667 | 3,9 | 4,6 | 4,7 | 4,7 |
| 2 | 25 %Softisan 649, 75 % Softisan 378) | 9,1 | 0,D907 | 1,9 | 4,2 | - | - |
| 3 | 100 % PEG 400, {Anzahl Einheiten, n = 9) | 218,7 | 2, 1867 | | | | |
| 4 | 100 % PEG 600, (Anzahl Einheiten, n = 14) | 186,7 | 1,8667 | | | | |
| 5 | 100 % PEG 1000, (Anzahl Einheiten, n = 23) | 256,0 | 2,5600 | | | | |
| 6 | 100 % PEG 2000, (Anzabl Einheiten, n = 46) | 314,7 | 3,1467 | | | | |
| 7 | 100 % Lusplan DD-DA5 | 80,0 | 0,8000 | | | | |
| B | 90 % Softisan 649, 10% PEG 1000 | 5,3 | 0,0533 | | | | |
| 9 | 90 % Softisan 649, 10% PEG 2000 | 16,5 | 0,1653 | | | | |
| 11 | 75 % Softisan 649, 25% PEG 400 | 4,8 | 0, D480 | | | | |
| 10 | 80 % Softisan 649, 10% Softisan 378, 10% PEG 1000 | 42,7 | 0,4267 | | | | |
| 12 | 25 % Softisan 649, 65% Softisan 378, 10% PEG 2000 | 43,7 | 0,4373 | 1,6 | 4,7 | 4,9 | 4,9 |
| 13 | 68,2 % Vaseline, 29,8 % Softisan 649, 2 % Tecerowachs | < 2 | | 0,3 * | 0,5 * | | |
| 14 | J&J, Actisorb | 40,6 | 0,4060 | 2,2 | 4,1 | | |
| 15 | S&N, Actiooat | 9530,0 | 95,3 | | | | |
| 16 | Coloplast, Contreet H | 1330 | 13,3 | | | | |
| 17 | Convatec, Aquacell Ag | 664 | 6,64 | | | | |

| | | | | | | | |
|---|---|---|---|---|---|---|---|
| * nach Methode AATCC 100-1999 | | | | | | | |

Die Erfindung soll im folgenden noch anhand einer Zeichnung weiter erläutert werden, dabei zeigen:
- Figur 1:: eine erste Ausgestaltung einer Wundauflage,
- Figur 2:: eine weitere Ausführungsform einer Wundauflage,
- Figur 3:: eine dritte Ausführungsform einer Wundauflage.

Die erste Figur zeigt eine erste Ausgestaltung einer erfindungsgemäßen Wundauflage umfassend einen Träger 1 aus einem Nylongewirke. Auf den Träger 1 ist auf seiner einer zu behandelnden Wunde 4 zugewandten Seite eine metallische Beschichtung 3 aufgebracht, die eine antimikrobielle Wirkung besitzt und hier aus elementarem Silber besteht. Darüber hinaus ist auf der metallbeschichteten Seite des Trägers 1 eine Zubereitung 3 in Form einer Salbe oder Creme aufgetragen, insbesondere aufgestrichen oder als Beschichtung aufgebracht. Die Wunde 4 steht damit unmittelbar nur mit der Zubereitung 3 in Kontakt, die die Abgabe von Metallionen aus der metallischen Beschichtung 2 kontrolliert, indem sie den Kontakt von Flüssigkeit mit der Metallschicht 3 vermittelt. Eine derartige Wundauflage kann entweder mittels z.B. eines Verbandes auf der Wunde 4 fixiert werden oder sie können unmittelbar in eine Wunde 4 eintamponiert werden.

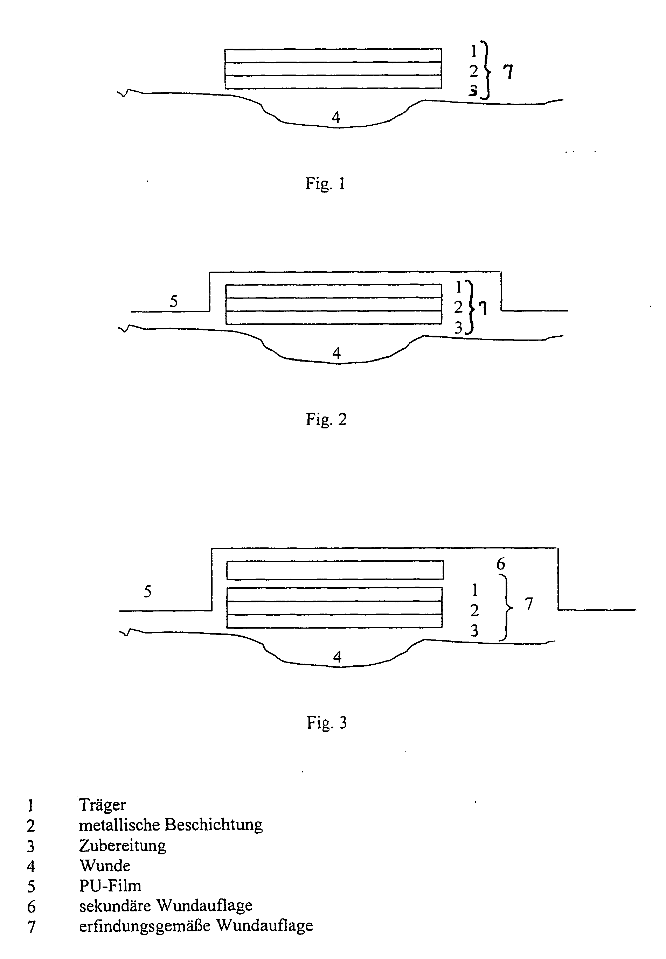

Figur 2 zeigt eine Wundauflage, die im wesentlichen der Wundauflage 7 (auch primäre Wundauflage 7) gemäß Figur 1 entspricht. Dabei sind gleiche Teile mit gleichen Bezugszeichen versehen. Zusätzlich zu der primären Wundauflage 7 gemäß Figur 1 ist hier noch eine Film 5 (Polyurethanfilm) vorgesehen, der auf der der Wunde 4 abgewandten Seite der primären Wundauflage 7 angebracht ist und diese im aufgebrachten Zustand auf die Haut eines Patienten vollständig überdeckt. Dabei ist der Film 5 mit der primären Wundauflage 7 über eine materialschlüssige Verbindung verbunden, insbesondere geklebt.

Desweiteren weist der Film 5 an seinen Randbereichen, die über die primäre Wundauflage 7 hinausragen, eine adhäsive Beschichtung dergestalt auf, dass die Wundauflage 7 auf der Haut eines Patienten befestigt werden kann. Als adhäsive Beschichtung kann dabei eine für adhäsive Wundauflagen übliche kontinuierliche oder diskontinuierliche Beschichtung ausgewählt werden. Die wesentlichen Parameter sind hierbei zum einen die Hautverträglichkeit (Wasserdampfdurchlässigkeit, ggf. Wasserdichtheit, Atmungsaktivität, Antiallergenztät, gute Ablösbarkeit) und zum anderen die Klebekraft (sicherer Halt). Die Wundauflage 7 wird dabei so gewählt hinsichtlich ihrer Abmessungen, dass die adhäsive Beschichtung nur auf unverletzter Haut zu liegen kommt.

Figur 3 zeigt nun eine weitere Ausgestaltung, wobei auch hier gleiche Teile mit gleichen Bezugszeichen versehen sind. Zusätzlich zu der primären Wundauflage 7 ist hier eine sekundäre Wundauflage 6 vorgesehen, die auf einer zweiten, der Wunde 4 abgewandten Seite des Trägers 1 angeordnet ist und zusätzliche Aufgaben bei der Wundversorgung, wie insbesondere Polsterung, aber auch Flüssigkeitsabsorption erfüllen kann.

Auf der der Wunde 4 abgewandten Seite der sekundären Wundauflage ist wiederum wie in Figur 2 ein Polyurethan-Film angebracht, der die gesamte Wundauflage, umfassend die primäre und die sekundäre Wundauflage überspannt und gegen die Umgebung abdeckt im applizierten Zustand.

## Patentansprüche

1. Wundauflage umfassend einen Träger (1) mit einer ersten einer zu behandelnden Oberfläche zugewandten Seite und einer zweiten gegenüberliegenden Seite, der mit einem antimikrobiell wirkenden Metall (2) ausgerüstet ist, wobei der Träger (1) mindestens auf seiner ersten Seite eine Zubereitung (3) zur lokalen Therapie umfasst, **dadurch gekennzeichnet, dass** die Zubereitung (3) eine Salbe oder Creme ist.

2. Wundauflage nach Anspruch 1, **dadurch gekennzeichnet, dass** die Zubereitung (3) wasserfrei ist.

3. Wundauflage nach einem der vorangehenden Ansprüche,
**dadurch gekennzeichnet, dass** das Metall als Beschichtung auf dem Träger (1) aufgebracht oder auf bzw. in den Träger (1) imprägniert ist.

4. Wundauflage nach einem der vorangehenden Ansprüche,
**dadurch gekennzeichnet, dass** das antimikrobiell wirkende Metall auf die erste Seite des Trägers (1) aufgebracht ist.

5. Wundauflage nach einem der vorangehenden Ansprüche,
**dadurch gekennzeichnet, dass** das Metall elementares Silber ist.

6. Wundauflage nach einem der vorangehenden Ansprüche,
**dadurch gekennzeichnet, dass** der Träger (1) ein Polyamidgewirke, insbesondere ein Nylongewirke ist.

7. Wundauflage nach einem der vorangehenden Ansprüche,
**dadurch gekennzeichnet, dass** der Träger (1) auf der ersten Seite mit der Zubereitung (3) beschichtet oder bestrichen ist.

8. Wundauflage nach einem der vorangehenden Ansprüche,
**dadurch gekennzeichnet, dass** die Wundauflage (7) weniger als 500 µg/ 100 cm² in 24h bei 37°C in 100 ml Wasser an antimikrobiellem Metall freisetzt.

9. Wundauflage nach Anspruch 8, **dadurch gekennzeichnet, dass** die Wundauflage (7) weniger als 400 µg/ 100 cm² in 24h bei 37°C in 100 ml Wasser an antimikrobiellem Metall freisetzt.

10. Wundauflage nach Anspruch 8 oder 9, **dadurch gekennzeichnet, dass** die Wundauflage (7) mind. 2 µg/ 100 cm² in 24h bei 37°C in 100 ml Wasser und weniger als 300 µg/ 100 cm² in 24h bei 37°C in 100 ml Wasser an antimikrobiellem Metall freisetzt.

11. Wundauflage nach einem der vorangehenden Ansprüche, **dadurch gekennzeichnet, dass** die Zubereitung (3) mindestens ein Mittel ausgewählt aus der Gruppe der Mono- , Di- oder Triglyceride, Fettsäureester Oligomerer des Glycerins, Fettalkohole, Fettsäureester, ethoxylierten Fettalkohole, ethoxylierten Fettsäuren, Polyethoxylen-Derivaten oder dimerisierten Fettsäureester oder Derivate hiervon umfasst.

12. Wundauflage nach Anspruch 11, **dadurch gekennzeichnet, dass** die Zubereitung (3) mindestens ein wasserbindendes Mittel umfasst.

13. Wundauflage nach Anspruch 11, **dadurch gekennzeichnet, dass** die Zubereitung (3) ein Gemisch aus Mono-, Di- und Triglyceriden und/oder Fettsäureester Oligomerer des Glycerins umfasst.

14. Wundauflage nach Anspruch 11, **dadurch gekennzeichnet, dass** die Zubereitung (3) mindestens ein wasserbindendes Mittel und dimerisierte Fettäureester umfasst.

15. Wundauflage nach einem der vorangehenden Ansprüche, **dadurch gekennzeichnet, dass** der Träger (1) mehrschichtig ist.

16. Wundauflage nach einem der vorangehenden Ansprüche,
**dadurch gekennzeichnet, dass** die Zubereitung (3) nicht vollflächig aufgetragen ist.

17. Verfahren zur Herstellung einer antimikrobiellen Wundauflage nach einem der vorangehenden Ansprüchen umfassend die Schritte (a) Bereitstellen eines mit antimikrobiell wirkenden Metall beschichteten Trägers und (b) Aufbringen einer Zubereitung zur lokalen Therapie, in Form einer Salbe oder Creme auf mindestens eine Seite des Trägers.

18. Verfahren nach Anspruch 17, **dadurch gekennzeichnet, dass** der Träger mit der Zubereitung beschichtet oder bestrichen wird.

19. Verwendung einer Wundauflage nach einem der Ansprüche 1 bis 16, umfassend einen mit einem antimikrobiell wirkenden Metall beschichteten Träger und eine auf mindestens einer Seite des Trägers aufgebrachte Zubereitung zur Herstellung eines Mittels zur Behandlung von Wunden.

## Claims

1. Wound dressing comprising a support (1) with a first side facing a surface to be treated and a second opposing side, the support (1) having an antimicrobially effective metal (2), wherein at least on its first side, the support (1) comprises a preparation (3) for local treatment, **characterized in that** the preparation (3) is an ointment or a cream.

2. Wound dressing according to claim 1, **characterized in that** the preparation (3) contains no water.

3. Wound dressing according to any one of the preceding claims, **characterized in that** the metal is applied to the support (1) in the form of a coating or is impregnated onto or into the support (1).

4. Wound dressing according to any one of the preceding claims, **characterized in that** the antimicrobially effective metal is disposed onto the first side of the support (1).

5. Wound dressing according to any one of the preceding claims, **characterized in that** the metal is elementary silver.

6. Wound dressing according to any one of the preceding claims, **characterized in that** the support (1) is a knitted polyamide fabric, in particular, a knitted nylon fabric.

7. Wound dressing according to any one of the preceding claims, **characterized in that** the preparation (3) is coated or spread on the first side of the support (1).

8. Wound dressing according to any one of the preceding claims, **characterized in that** the wound dressing (7) releases less than 500µg/100 cm² of antimicrobial metal in 24 h at 37°C in 100 ml water.

9. Wound dressing according to claim 8, **characterized in that** the wound dressing (7) releases less than 400µg/100 cm² of antimicrobial metal in 24 h at 37°C in 100 ml water.

10. Wound dressing according to claim 8 or 9, **characterized in that** the wound dressing (7) releases at least 2µg/100 cm² of antimicrobial metal in 24h at 37°C in 100 ml water and less than 300 µg/100 cm² in 24h at 37°C in 100 ml water.

11. Wound dressing according to any one of the preceding claims, **characterized in that** the preparation (3) comprises at least one agent selected from the group of mono-, di- or triglycerides, fatty acid ester oligomer of glycerine, fatty alcohols, fatty acid ester, ethoxylated fatty alcohols, ethoxylated fatty acids, polyethoxylene derivatives or dimerised fatty acid esters or derivatives thereof.

12. Wound dressing according to claim 11, **characterized in that** the preparation (3) comprises at least one water- bonding agent.

13. Wound dressing according to claim 11, **characterized in that** the preparation (3) comprises a mixture of mono-, di- and triglycerides and/or fatty acid ester oligomer of the glycerine.

14. Wound dressing according to claim 11, **characterized in that** the preparation (3) comprises at least one water- bonding agent and dimerised fatty acid ester.

15. Wound dressing according to any one of the preceding claims, **characterized in that** the support (1) has several layers.

16. Wound dressing according to any one of the preceding claims, **characterized in that** the preparation (3) is not applied to the full surface.

17. Method for producing an antimicrobial wound dressing according to any one of the preceding claims, comprising the steps (a) providing a support coated with an antimicrobially effective metal and (b) applying a preparation for local therapy, in form of an ointment or cream onto at least one side of the support.

18. Method according to claim 17, **characterized in that** the preparation is coated or spread onto the support.

19. Use of a wound dressing according to any one of the claims 1 through 16, comprising a support coated with an antimicrobial metal and a preparation which is applied to at least one side of the support, for producing an agent for the treatment of wounds.

## Revendications

1. Pansement comprenant un support (1) avec un premier côté orienté vers la surface à traiter et un deuxième côté opposé, muni d'un métal à action antimicrobienne (2), le support (1) comprenant au moins sur son premier côté une préparation (3) pour le traitement local, **caractérisé en ce que** la préparation (3) est une pommade ou une crème.

2. Pansement selon la revendication 1, **caractérisé en ce que** la préparation (3) est anhydre.

3. Pansement selon l'une des revendications précédentes, **caractérisé en ce que** le métal est appliqué comme revêtement sur le support (1) ou bien est imprégné dans le support (1).

4. Pansement selon l'une des revendications précédentes, **caractérisé en ce que** le métal à action antimicrobienne est appliqué sur le premier côté du support (1).

5. Pansement selon l'une des revendications précédentes, **caractérisé en ce que** le métal est de l'argent élémentaire.

6. Pansement selon l'une des revendications précédentes, **caractérisé en ce que** le support (1) est un maillage en polyamide, en particulier un maillage en nylon.

7. Pansement selon l'une des revendications précédentes, **caractérisé en ce que** le support (1) est recouvert ou enduit sur le premier côté de la préparation (3).

8. Pansement selon l'une des revendications précédentes, **caractérisé en ce que** le pansement (7) libère moins de 500 µg/100 cm² de métal antimicrobien en 24 h à 37°C dans 100 ml d'eau.

9. Pansement selon la revendication 8, **caractérisé en ce que** le pansement (7) libère moins de 400 µg/100 cm² de métal antimicrobien en 24 h à 37°C dans 100 ml d'eau.

10. Pansement selon la revendication 8 ou 9, **caractérisé en ce que** le pansement (7) libère au moins 2 µg/100 cm² de métal antimicrobien en 24 h à 37°C dans 100 ml d'eau et moins de 300 µg/100 cm² de métal antimicrobien en 24 h à 37°C dans 100 ml d'eau.

11. Pansement selon l'une des revendications précédentes, **caractérisé en ce que** la préparation (3) comprend un agent choisi parmi le groupe constitué des mono-, di- ou triglycérides, oligomères d'esters d'acides gras de la glycérine, alcools gras, esters d'acides gras, alcools gras éthoxylés, dérivés de polyéthoxylène ou esters d'acides gras dimérisés ou bien des dérivés de ces derniers.

12. Pansement selon la revendication 11, **caractérisé en ce que** la préparation (3) comprend au moins un agent qui absorbe l'eau.

13. Pansement selon la revendication 11, **caractérisé en ce que** la préparation (3) comprend un mélange de mono-, di- et triglycérides et/ou des oligomères d'esters d'acides gras de la glycérine.

14. Pansement selon la revendication 11, **caractérisé en ce que** la préparation (3) comprend au moins un agent retenant l'eau et des esters d'acides gras dimérisés.

15. Pansement selon l'une des revendications précédentes, **caractérisé en ce que** le support (1) a plusieurs couches.

16. Pansement selon l'une des revendications précédentes, **caractérisé en ce que** la préparation (3) n'est pas appliquée sur toute la surface.

17. Procédé pour fabriquer un pansement antimicrobien selon l'une des revendications précédentes comprenant les étapes consistant à (a) préparer un support revêtu d'un métal à action antimicrobienne et (b) appliquer une préparation en vue d'un traitement local, sous forme d'une pommade ou d'une crème, sur au moins un côté du support.

18. Procédé selon la revendication 17, **caractérisé en ce que** le support est recouvert ou enduit de la préparation.

19. Utilisation d'un pansement selon l'une des revendications 1 à 16, comprenant un support recouvert d'un métal à action antimicrobienne et une préparation appliquée sur au moins un côté du support, pour la fabrication d'un moyen pour traiter des plaies.
